Europäisches Patentamt

European Patent Office

Office européen des brevets

(11) Publication number: **0 310 900 B1**

## EUROPEAN PATENT SPECIFICATION

(45) Date of publication of patent specification: **02.12.92**  (51) Int. Cl.5: **B65B 55/10**, A61L 2/20

(21) Application number: **88115762.2**

(22) Date of filing: **24.09.88**

(54) **An arrangement for the sterilizing of a travelling material web.**

(30) Priority: **09.10.87 SE 8703903**

(43) Date of publication of application:
**12.04.89 Bulletin 89/15**

(45) Publication of the grant of the patent:
**02.12.92 Bulletin 92/49**

(84) Designated Contracting States:
**AT BE CH DE ES FR GB IT LI NL SE**

(56) References cited:
**SE-B- 304 082
US-A- 3 812 598
US-A- 4 274 210
US-A- 4 603 490**

(73) Proprietor: **ROBY TEKNIK AB
Box 61
S-221 00 Lund(SE)**

(72) Inventor: **Olanders, Pär
Rudeboksvägen 828
S-222 55 Lund(SE)**

(74) Representative: **Sundell, Hakan
AB Tetra Pak Ruben Rausings gata
S-221 86 Lund(SE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

# Description

The present invention relates to an arrangement for the sterilizing of a travelling material web, e.g. of the type which afterwards is to be converted to so-called aseptic packages, this arrangement comprising a chamber with inlet and outlet for the web and also inlet and outlet openings for a sterilizing gas flow.

An arrangement of this general type for treatment of a moving web with an air flow for drying the web is known through US-A-4 603 490. This known arrangement comprises a chamber including an inlet and an outlet for the flow of air, an inlet and outlet for the material web and a baffle plate disposed over the air outlet at an angle in relation to the path of travel of the web through the chamber.

Consumer packages of a non-returnable character are manufactured at present with the help of modern, high-capacity machines of the type which from a web or from prefabricated blanks of a packing material form, fill and close the packages. The machines manufacture packages from a web by first converting the web to a tube by durably joining together the longitudinal edge zones in a strong overlap joint. The tube formed is then filled with the intended contents and divided into individual, filled packing units through repeated transverse sealings of the tube across the longitudinal axis of the tube below the actual contents level, whereafter the packing units are separated from one another by means of transverse cuts in the transverse sealings made, and, possibly after a final shaping, discharged from the machine as finished packages.

A very large group of these so-called non-returnable packages is manufactured from a material comprising a carrier layer of paper or cardboard and outer and inner coatings of plastics, in particular thermoplastics, which beside making the packages liquid-tight also may he used for performing the said sealings through so-called heat-sealing during the manufacture of the packages.

With the help of packing machines of the said type it is also known to make so-called aseptic packages for certain types of sensitive, liquid foodstuffs,e.g. milk, in order to prolong the keeping properties of the contents. The aseptic machines operate in principle in the same manner as the machines described earlier, but with the important difference that the manufacture of the packages is carried out under aseptic conditions which means that the contents as well as the packing material have to be sterile, and likewise the atmosphere in the machine wherein the tubes are formed and filled. The sterile atmosphere in the machine is obtained in that a certain pressure of sterile gas, usually superheated sterile air, is maintained inside the tubes as well as in the close environment of the tubes, as a result of which leakage of polluted, non-sterile air from the outer environment of the machine is prevented. The sterile contents usually are obtained by subjecting the contents prior to filling to a heat treatment whereby the contents for a certain period are heated to, and held at, a sufficiently high temperature in order to eliminate harmful micro-organisms. It has been easy as a rule to fulfil these two sterility requirements, but it has been found more difficult, with the methods available up to now, to provide a simple, effective sterilization of the weblike packing material.

The sterilizing of the packing material web is carried out in a known realization by passing the web prior to conversion to tubular shape through a bath of chemical sterilizing agent. usually a 10-35% hydrogen peroxide solution which is made to moisten the packing material, whereupon the surplus of liquid is removed from the web by means of calender cylinders. Any sterilizing agent remaining on the web is removed, after conversion of the web to a tube, by a heating arrangement which heats the material tube to such a degree that the agent is evaporated and driven off through the upper, open end of the tube.

In accordance with another known method the packing material web is passed through a chamber containing heated, gaseous sterilizing agent, preferably a mixture of hydrogen peroxide and steam, to absorb hydrogen peroxide through condensation on the material web. In this known method too the remaining sterilizing agent is removed by evaporation.

Even though the known methods described here, which use liquid sterilizing agent either directly or indirectly through condensation, function well for material webs with plain, uniform surfaces, it has proved more difficult to achieve an effective sterilization of packing material webs with surface irregularities, e.g. tear strips (so-called pull-tabs) sealed over prepared emptying openings. This is due, at least partly, to the material web being in contact with the sterilizing agent during a time which is too short to allow the sterilizing agent to penetrate, and act in,the less readily accessible spaces of such irregularities. Another problem, which is also connected with using the sterilizing agent in liquid form, and which becomes particularly manifest when the web is passed through a bath, is the difficulty of preventing a so-called edge absorption of sterilizing agent in web portions with freely exposed fibre layers, e.g. in the area along longitudinal cutting edges of the web which easily absorb moisture.

It is known that a mixture of hydrogen peroxide and water in gas form has a sterilizing effect which increases with rising temperature, and it is known, moreover, that gas, by contrast to liquid, can easily penetrate into less readily accessible areas of the type which occur on material webs with surface irregularities, and a natural and obvious solution of the problem which are inherent in the known methods described should be, therefore, to substitute the sterilizing agent in liquid form by a corresponding sterilizing agent in gas form and carry out the sterilization exclusively in the gas phase, that is to say without condensation.

In spite of these theoretical prerequisites for an effective sterilization to be carried out with the help of gaseous sterilizing agent, e.g. water/hydrogen peroxide - vapour,regardless of the surface quality of the material web, it has been difficult up to now to realize this method.

It is the object of the present invention,therefore,to indicate an arrangement by means of which such a gas phase sterilization of a travelling material web is practically possible.

This object is achieved in accordance with the present invention in that an arrangement of the type described in the introduction is given the characteristic that the chamber has constriction zones arranged between the inlet and the outlet which are connected to one another through an intermediate chamber portion and through which constriction zones a web of packaging material, of the type used for producing aseptic packages, can only just pass freely, that the intermediate chamber portion is connected to a source of the sterilizing gas by means of at least one inlet opening provided in the intermediate chamber portion, and that the constriction zones are connected to an external vacuum source through one or more outlet openings for the gas arranged adjacent to and outwardly of the constriction zones.

Owing to the zones of the chamber being designed as constrictions between the inlet and outlet of the material web the gas entering through the inlet opening or openings in the intermediate chamber portion is forced to flow at very high speed in close contact with the material web passing by within the areas of the said constriction zones, which ensures good contact and consequently effective sterilizing action uniformly distributed over the whole width of the material web.

The intermediate chamber portion serving as a distributing space for the incoming sterilizing gas preferably has a somewhat larger free transverse flow area than the constriction zones situated on either side of the chamber so as to facilitate and ensure an effective distribution of the sterilizing agent on both sides of the material web. However, in accordance with the invention the intermediate chamber portion may also be designed, with the same transverse flow area as the constriction zones and in fact constitute the intermediate part of a single unbroken constriction zone, the good distribution of the sterilizing gas aimed at being provided in this case with the help of oppositely directed inlet openings in the intermediate chamber part, preferably arranged on either side of the material web.

In accordance with a simple embodiment of the invention the chamber is arranged in an elongated, rectangular box with the inlet and outlet of the material web located straight before one another at opposite ends of the box, as a result of which the material web can he conducted through the chamber without coming into contact with any parts of the chamber. The inlet opening or openings for the sterilization gas preferably are arranged here in a chamber portion situated centrally between the inlet and the outlet for the web. The constriction zones may be formed, for example, by elongated restricting elements or plates, situated oppositely in pairs and arranged between the intermediate chamber portion and the inlet and the outlet respectively, which between them form narrow, gaplike passages of a design which is such that the web can only just pass freely. The plates or corresponding passage-limiting elements, which may be suspended or fixed to form a seal in some other appropriate manner on the inner walls of the box, are preferably arranged with their remote ends situated at some distance from the neighbouring end walls of the box so as to form end chamber portions located at the inlet and outlet respectively with a larger free flow passage than the constriction zones or passages formed between the plates, the outlet openings for the sterilizing gas being arranged in direct connection to these end portions. Thanks to such a location of the outlet openings a virtually complete evacuation from the constriction zones is facilitated and assured.

When the arrangement in accordance with the invention is to be used for the sterilization of a material web intended for the manufacture of aseptic packing containers, the sterilizing gas as well as the material web have to be heated and maintained at a temperature above the dew point of the sterilizing gas so as to avoid any condensation of the gas on the material web. To assure such condensation-free sterilization the arrangement in accordance with the invention may comprise a heating arrangement placed upstream of and/or immediately adjacent to the chamber through which, or past which, the material web is conducted for heating before entry into the chamber. Preferably the chamber is also provided with a suitable heating arrangement, e.g. electric heating elements and/or a source of radiation e.g. UV-light, which also provides the possibility of an improved sterilizing effect through synergism, arranged around or within the walls of the chamber,so as to heat the chamber walls to a sufficient extent in order to eliminate the risk of condensation occurring on the inner wells of the chamber

As mentioned earlier the outlet openings for the sterilizing gas are connected to an external vacuum source with the help of which the rapid gas flow through the chamber is achieved. The vacuum source may comprise, for example, a so-called water ring compressor or some other suitable pressure-reducing system by means of which the sterilizing gas can be purified before reutilization.

The invention will be described in greater detail with special reference to the attached drawing, wherein

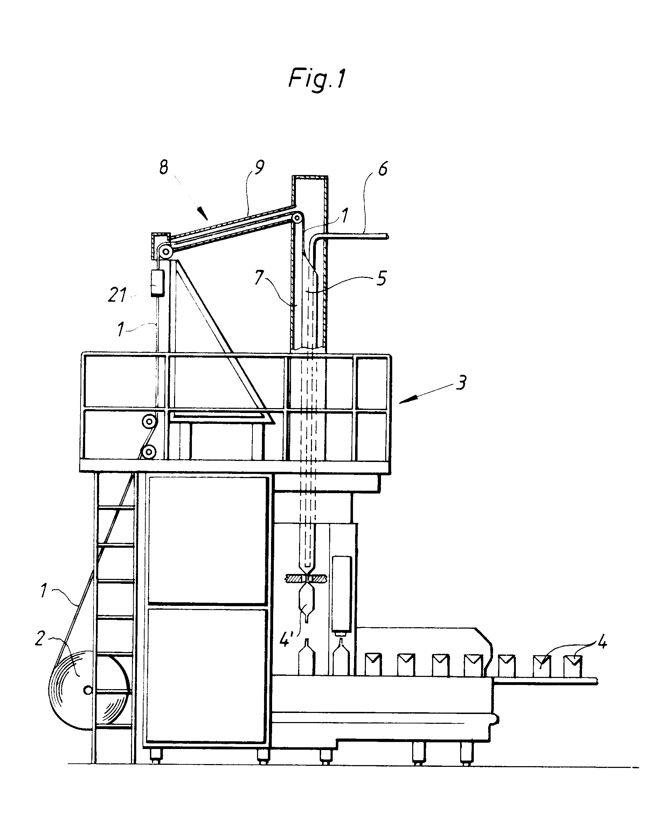

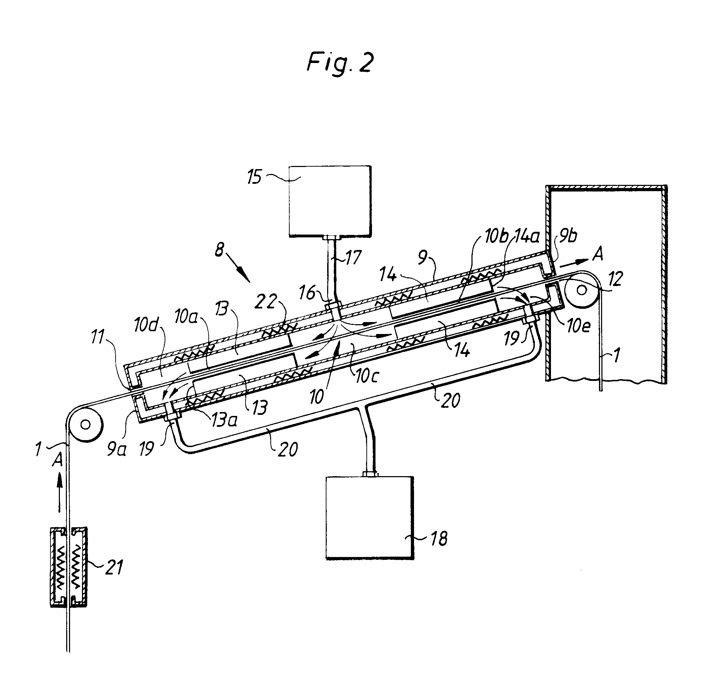

Fig.1 show how an arrangement in accordance with the invention can be installed and used in a conventional packing machine of the type which manufactures aseptic packing containers from a web of packing meterial and Fig.2 illustrates more closely the arrangement in accordance with the invention.

From a material web 1, which is supplied from a magazine roll 2, the packing machine shown (with the general designation 3) manufactures finished, filled packages 4 in that the web 1 first is converted to a tube 5 by durably joining together the longitudinal edges of the web in a longitudinal overlap joint. The tube 5 is filled with the intended contents through a filling pipe 6 introduced through the upper open end of the tube and is divided into individual packing units 4' through repeated transverse sealings across the longitudinal axis of the tube below the actual contents level in the tube, whereafter the packing units 4' are separated from one another by bans of cuts in the transverse sealings made. The packing units 4' are then conducted through a final shaping station in the machine and discharged thereafter as finished packages 4 at the discharge end of the machine.

It is a prerequisite, if the packages are to be aseptic, that the contents to be packed and the material web 1 must be sterile, and that the whole filling process including the conversion of the web to a tube in the filling zone 7 of the machine is carried out in a sterile environment. The sterility of the contents is achieved by subjecting the contents prior to filling to a heat treatment according to a previously specified temperature/time scheme, and the sterile environmental atmosphere in the filling zone 7 is provided by maintaining a certain pressure of hot sterile air within this zone, as a result of which leakage of polluted, non-sterile air iron the environment of the machine is prevented. The sterility of the material web 1 which is fed into the filling zone 7 of the machine through a sealed inlet at the upper end of the zone is provided with the help of the arrangement in accordance with the invention (generally designated 6) shown at the top of the machine.

The arrangement 8 in accordance with the invention has an elongated, rectangular box 9 arranged at the top of the packing machine 3 comprising a chamber 10 with inlet 11 and outlet 12 for the material web 1 located straight before one another at opposite ends 9a and 9b respectively of the box 9. The box 9 which may be made, for example, from stainless steel here has plate elements 13 and 14 respectively situated oppositely in pairs which are fixed so as to form a seal to the inner walls of the chamber 10 and designed so that in pairs between themselves they form gaplike passages or constriction zones 10a and 10b respectively situated straight before one another.The constriction zones 10a and 10b are dimensioned so that the material web 1 can only just pass freely, and are connected to one another by a chamber portion 10c located in between. The ends 13a and 14a of the plates 13 and 14 facing away from one another are arranged to terminate at a short distance from the end walls 9a and 9b respectively of the box so as to form inner chamber portions 10d and 10e respectively situated at corresponding ends of the box 9. As is evident, the constriction zones 10a and 10b have a much smaller free transverse flow area than the end portions 10d and 10e and the intermediate portion 10c of the chamber 10.

The intermediate chamber portion 10c is connected to an external source 15 of sterilizing gas through an inlet opening 16 arranged in the chamber portion 10c and a duct 17 connected thereto, whilst the end portions 10d and 10e of the chamber are in connection with an external vacuum source 18 through outlet openings 19 arranged in respective end portions and ducts 20 connected to them.

The vacuum source 18 consists preferably of a so-called water-ring compressor or a corresponding pressure-reducing means which make possible a regeneration of the sterilizing gas flowing out through the outlet openings 19.

The arrangement 8 in accordance with the invention, moreover, has a heating arrangement 21 placed upstreams of the inlet 11 of the chamber 10, with the help of which the material web 1 travelling past, or through, the heating arrangement 21 prior to entry into the box 9 can be heated to a temperature above the dew point of the sterilization gas used so as to prevent condensation of the sterilization gas on the material web 1 when the same passes through the box 9. In or around the walls of the box 9 are provided, moreover, electrically heated heating elements 22 for heating the inner walls of the chamber, and thereby avoiding the formation of condensation on the same.

The arrangement 8 functions in the following manner: When the material web 1, preheated with the help of the heating arrangement 21, which is to be sterilized by means of the arrangement 8, is fed in direction of the arrow A via a deflection roller into and through the chamber 10 it is brought into intimate contact with the sterilizing gas flowing in through the inlet opening 16 in the intermediate chamber portion 10c which in the intermediate chamber portion 10c distributes itself well on both sides of the material web and which has forced upon it through the vacuum source 18 connected to the outlet openings 19 a very high flow velocity in close contact with the two sides of the material web in the constriction zones 10a and 10b formed between the plates 13 and 14 respectively, as a result of which good contact is achieved within these constricted chamber portions between the sterilizing gas and the material web passing by along the whole width of the web. After passage through the constriction zones 10a and 10b the sterilizing gas is evacuated from the chamber 10 through the outlet openings 10 and the ducts 20 for possible regeneration and reutilization. The sterilized material web 1 is conducted out through the outlet opening 12 of the chamber and further into, and through, the sterile filling zone 7 of the packing machine 3 shown in Fig.1 for conversion to aseptic packages 4.

The arrangement in accordance with the invention described above can be used in principle for the sterilization of any conceivable travelling material web, but has been found to function especially well in the sterilization of laminated packing material of the type mentioned earlier for conversion to aseptic packing containers. With the help of the arrangement in accordance with the invention it has thus proved to be possible, in a simple manner, to achieve an effective sterilization of a packing material web, irrespective of the surface structure of the material web.

## Claims

1. An arrangement for the sterilizing of a travelling material web (1), this arrangement comprising a chamber (10) with inlet (11) and outlet (12) for the web and also inlet and outlet openings (16 and 19 respectively) for a sterilizing gas flow, **characterized in that** the chamber has constriction zones (10a and 10b) arranged between the inlet (11) and the outlet (12) which are connected to one another through an intermediate chamber portion (10c) and through which constriction zones a web of packaging material, of the type used for producing aseptic packages, can only just pass freely, that the intermediate chamber portion (10c) is connected to a source of the sterilizing gas (15) by means of at least one inlet opening (16) provided in the intermediate chamber portion (10c), and that the constriction zones (10a and 10b) are connected to an external vacuum source (18) through one or more outlet openings (19) for the gas arranged adjacent to and outwardly of the constriction zones.

2. An arrangement in accordance with claim 1, **characterized in that** the chamber (10) comprises elongated constriction elements (13 and 14 respectively) situated oppositely in pairs which between them delimit elongated gaplike spaces with reduced free flow area so as to form the constriction zones (10a and 10b).

3. An arrangement in accordance with claim 2, **characterized in that** remote ends (13a and 14a respectively) of the constriction elements (13 and 14) are arranged at a short distance from the oppositely situated end walls (9a and 9b respectively) of the chamber so as to form corresponding chamber portions (10d and 10e respectively) arranged at the inlet (11) and outlet (12) of the chamber within which are provided the said outlet openings (19).

4. An arrangement in accordance with anyone of the preceding claims, **characterized in that** the arrangement also comprises a heating arrangement (21) placed upstream of the chamber inlet (11) through which, or past which, the material web (1) is adapted to be conducted so as to be heated before, and/or in conjunction with, the entry into the chamber (10).

5. An arrangement in accordance with anyone of the preceding claims, **characterized in that** the walls of the chamber (1) comprise electrically heated heating elements (22) and/or a source of radiation , e.g. UV-light.

## Patentansprüche

1. Vorrichtung zum Sterilisieren einer laufenden Materialbahn (1), wobei die Vorrichtung eine Kammer (10) aufweist mit einem Einlaß (11) und einem Auslaß (12) für die Bahn und ferner Einlaß- und Auslaßöffnungen (16 bzw. 19) für den Durchfluß von Sterilisationsgas, **dadurch gekennzeichnet,** daß die Kammer Verengungszonen (10a und 10b) hat, die zwischen dem Einlaß (11) und dem Auslaß (12) angeordnet sind und die miteinander durch einen Kammerzwischenbereich (10c) verbunden sind und durch die eine Packstoffbahn der zur Herstellung von aseptischen Packungen verwendeten Art gerade eben ungehindert laufen kann, daß der Kammerzwischenbereich (10c) mit einer Quelle des Sterilisationsgases (15) über wenigstens eine Einlaßöffnung (16) verbunden ist, die in dem Kammerzwischenbereich (10c) vorgesehen ist, und daß die Verengungszonen (10a und 10b) mit einer externen Vakuumquelle (18) über ein oder mehr Auslaßöffnungen (19) für das Gas, die nahe den Verengungszonen und außerhalb derselben angeordnet sind, verbunden sind.

2. Vorrichtung nach Anspruch 1,
**dadurch gekennzeichnet,**
daß die Kammer (10) langgestreckte Verengungselemente (13 bzw. 14) aufweist, die paarweise einander gegenüberstehend positioniert sind und zwischen sich langgestreckte spaltartige Zwischenräume mit verringertem freiem Strömungsquerschnitt begrenzen, um die Verengungszonen (10a und 10b) zu bilden.

3. Vorrichtung nach Anspruch 2,
**dadurch gekennzeichnet,**
daß ferne Enden (13a bzw. 14a) der Verengungselemente (13 und 14) in einem geringen Abstand von den einander gegenüberliegenden Endwänden (9a bzw. 9b) der Kammer angeordnet sind, um entsprechende Kammerbereiche (10d bzw. 10e) zu bilden, die am Einlaß (11) und am Auslaß (12) der Kammer angeordnet sind und in denen die Auslaßöffnungen (19) vorgesehen sind.

4. Vorrichtung nach einem der vorhergehenden Ansprüche,
**dadurch gekennzeichnet,**
daß die Vorrichtung ferner eine Heizeinrichtung (21) aufweist, die an der Aufstromseite des Kammereinlasses (11) angeordnet ist und durch die oder an der vorbei die Materialbahn (1) geleitet wird, um vor und/oder in Verbindung mit dem Eintritt in die Kammer (10) aufgeheizt zu werden.

5. Vorrichtung nach einem der vorhergehenden Ansprüche,
**dadurch gekennzeichnet,**
daß die Wände der Kammer (1) elektrisch beheizte Heizelemente (22) und/oder eine Strahlungsquelle wie beispielsweise UV-Licht aufweisen.

**Revendications**

1. Dispositif pour la stérilisation d'une feuille de matière en déplacement (1), ce dispositif comprenant une chambre (10) avec une entrée (11) et une sortie (12) pour la feuille et également des orifices d'entrée et de sortie (16 et 19 respectivement) pour un flux de gaz de stérilisation, caractérisé en ce que la chambre comprend des zones d'étranglement (10a et 10b),situées entre l'entrée (11) et la sortie (12), qui sont reliées l'une à l'autre par une partie de chambre intermédiaire (10c), ces zones d'étranglement permettant seulement à une feuille de matière d'emballage, du type utilisé pour la fabrication d'emballages aseptiques, de passer juste librement ; en ce que la partie de chambre intermédiaire (10c) est raccordée à une source du gaz de stérilisation (15) par au moins un orifice d'entrée (16) prévu dans la partie de chambre intermédiaire (10c) ; et en ce que les zones d'étranglement (10a et 10b) sont raccordées à une source de vide extérieure (18) par un ou plusieurs orifices de sortie (19) pour le gaz, situés près et du côté extérieur des zones d'étranglement.

2. Dispositif suivant la revendication 1, caractérisé en ce que la chambre (10) comprend des éléments d'étranglement allongés (13 et 14 respectivement), placés en opposition en paires,qui délimitent entre eux des espaces allongés en forme de fente à section d'écoulement libre réduite, afin de définir les zones d'étranglement (10a et 10b).

3. Dispositif suivant la revendication 2, caractérisé en ce que les extrémités distantes (13a et 14a respectivement) des éléments d'étranglement (13 et 14) sont situées à une faible distance des parois d'extrémité opposées (9a et 9b respectivement) de la chambre, de manière à définir des parties de chambre correspondantes (10d et 10e respectivement) situées à l'entrée (11) et à la sortie (12) de la chambre et dans lesquelles sont prévus lesdits orifices de sortie (19).

4. Dispositif suivant l'une quelconque des revendications précédentes, caractérisé en ce que le dispositif comprend également des moyens de chauffage (21) placés en amont de l'entrée de chambre (11) et à travers lesquels, ou devant lesquels, on peut faire passer la feuille de matière (1) de façon à la chauffer avant, et/ou en combinaison avec l'entrée dans la chambre (10).

5. Dispositif suivant l'une quelconque des revendications précédentes, caractérisé en ce que les parois de la chambre (10) comportent des éléments chauffants électriques (22) et/ou une source de rayonnement, par exemple de lumière ultraviolette.

# Fig.1

*Fig. 2*